# EUROPEAN PATENT APPLICATION

(11) **EP 2 011 537 A1**
(43) Date of publication of application: **07.01.2009**
(21) Application number: 07111996.0
(22) Date of filing: 06.07.2007
(51) Int. Cl.: A61M 15/00

(54) **Inhaler**

(71) Applicant: Vectura Delivery Devices Limited, Chippenham Wiltshire SN14 6FH (GB)
(72) Inventor: Eason, Stephen William, Redgrave, Diss, Norfolk IP22 1RX (GB); Sarkar, Matthew, Cambridge, CB4 3JU (GB)
(74) Representative: Grey, Ian Michael

(57) **Abstract**

An inhaler is disclosed. It comprises a housing to receive an elongate strip of blisters each containing a dose of medicament and being sequentially movable into alignment with means for breaching a blister to enable a user to inhale said dose contained therein. The device comprises an unused and a used blister compartment separated by a wall which may be moulded integrally with the housing of the device.

## Description

The present invention relates to an inhalation device for oral or nasal delivery of medicament in powdered form and to an inhaler containing a strip of blisters each having a breachable lid and/or base that contains a dose of medicament for inhalation by a user of the device.

Oral or nasal delivery of a medicament using an inhalation device is a particularly attractive method of drug administration as these devices are relatively easy for a patient to use discreetly and in public. As well as delivering medicament to treat local diseases of the airway and other respiratory problems, they have more recently also been used to deliver drugs to the bloodstream via the lungs thereby avoiding the need for hypodermic injections.

It is common for dry powder formulations to be pre-packaged in individual doses, usually in the form of capsules or blisters which each contain a single dose of the powder which has been accurately and consistently measured. A blister is generally cold formed from a ductile foil laminate or a plastics material and includes a puncturable or peelable lid which is heat-sealed around the periphery of the blister during manufacture and after introduction of the dose into the blister. A foil blister is preferred over a polymer blister or gelatine capsule as each dose is protected from the ingress of water and penetration of gases such as oxygen in addition to being shielded from light and UV radiation all of which can have a detrimental effect on the delivery characteristics of the inhaler if a dose becomes exposed to them. Therefore, a blister offers excellent environmental protection to each individual drug dose.

Inhalation devices that receive a blister pack comprising a number of blisters each of which contain a pre-metered and individually packaged dose of the drug to be delivered are known. Actuation of the device causes a mechanism to breach or rupture a blister, such as by puncturing it or peeling the lid off, so that when the patient inhales, air is drawn through the blister entraining the dose therein that is then carried out of the blister through the device and via the patient's airway down into the lungs. Pressurized air or gas or other propellants may also be used to carry the dose out of the blister. Alternatively, the mechanism that punctures or opens the blister may also push or eject the dose out of the blister into a receptacle from which the dose may subsequently be inhaled.

It is advantageous for the inhaler to be capable of holding a number of doses to enable it to be used repeatedly over a period of time without the requirement to open and/or insert a blister into the device each time it is used. Therefore, many conventional devices include means for storing a number or strip of blisters each containing an individual dose of medicament. When a dose is to be inhaled, an indexing mechanism moves a previously emptied blister away from the opening mechanism so that a fresh one is moved into a position ready to be opened for inhalation of its contents.

An inhaler of the type described above is known from the Applicants own copending international application no. PCT/GB2004/004416 filed on 18^{th} October 2004 and claiming priority from GB application no. 0324358.1 filed 17^{th} October 2003. This international application has been published as WO 2005/037353 A1.

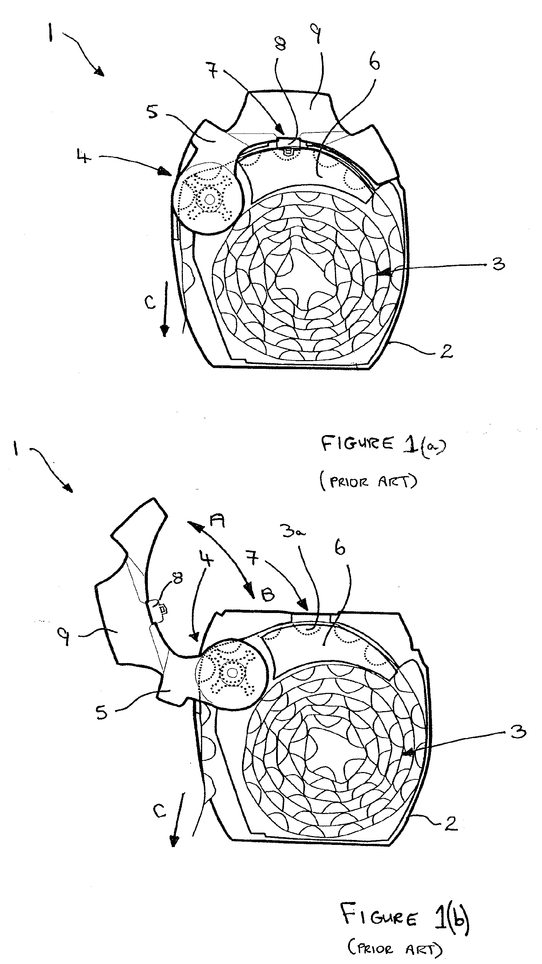

According to one embodiment described and claimed in WO 2005/037353 A1, and illustrated in Figures 1a and 1b of the accompanying drawings, an inhaler 1 has a housing 2 containing a coiled strip of blisters 3. An indexing mechanism 4 comprising a single actuating lever 5 unwinds the coil 3 one blister at a time so that they pass over a blister locator chassis 6 and successively through a blister piercing station 7, when the actuator 5 is pivoted in a direction indicated by arrow "A" in Figure 1b. The blister 3a located at the blister piercing station 7 on each movement of the actuator 5 is pierced on the return stroke of the actuator 5 (in the direction indicated by arrow "B" in Figure 1b) by piercing elements 8 on the actuator 5 itself so that, when a user inhales through a mouthpiece 9, an airflow is generated within the blister 3a to entrain the dose contained therein and carry it out of the blister 3a via the mouthpiece 9 and into the user's airway.

Although the inhalation device referred to above and described in the aforementioned publication has addressed many of the known problems associated with these types of devices, it is designed so as to store only a small number of used blisters within the device so that, when that number of blisters is exceeded, they extend out of the housing of the device so that the user must separate those used blisters from those unused blisters that remain within the device and discard the detached portion of the strip. The direction of movement of the used blisters is indicated by arrow "C" in Figures 1a and 1b. The blister strip 3 may be perforated or weakened between each or a number of blisters to facilitate the tearing of used blisters from the strip 3.

Although devices that eject used blisters have the advantage of being particularly small and lightweight, it is desirable to provide a fully integrated device in which all the used blisters are retained within the device so that separation of used blisters from those that remain in the device is no longer necessary. Not only would this make the device simpler to use because the user no longer has to concern themselves with periodic detachment and disposal of a used portion of the blister strip but any potential contamination of the fingers by residual drug remaining on the used blisters can be avoided because there is no need for the user to come into contact with any of the used blisters. Therefore, the entire strip can be effectively sealed within the housing of the device.

A potential complication with inhalation devices that retains used blisters is that a small amount of the powdered dose, typically between 1% - 5%, may remain in each blister after inhalation. Furthermore, if a patient indexes the strip without having previously inhaled the dose in a blister that has been pierced or breached, the amount of residual powder will be substantial. It is therefore important to prevent the unused blisters from becoming contaminated with loose powder that could have a detrimental effect on the operation of the device and also result in the patient exceeding an intended dose as they may inhale some of the residual powder as well as the contents of a pierced blister. Furthermore, if the residual powder has been exposed to the atmosphere, it may have also degraded making it unsuitable for inhalation.

The present invention seeks to provide an inhalation device that retains a used strip of blisters within the housing of the device. More specifically, the present invention seeks to address the problem of residual powder containment to prevent residual powder from contaminating unused blisters remaining in the device and from being inhaled by a user of the device.

Although the device may be disposable after all the blisters contained within it have been exhausted, it is envisaged that it may be possible to open the housing to enable the old strip to be removed and a fresh one inserted. It is also envisaged that blisters may be retained within a portion of the housing of the device which is detachable from the remainder of the housing in which the indexing and piercing mechanism is located, thereby forming a replaceable cartridge. This would enable an exhausted blister strip to be removed without direct contact by the patient.

According to one aspect of the invention, there is provided a housing to receive a strip of blisters each containing a dose of medicament and means to sequentially move each blister into alignment with means for opening a blister to enable a user to inhale said dose, the inhaler having a first compartment to contain unused blisters and a second compartment to receive used blisters, the first and second compartments being separated by a dividing wall.

The dividing wall is preferably a fixed dividing wall and may be formed integrally with the housing of the device.

Preferably, an aperture is provided in the dividing wall for the passage of the blister strip from the first compartment into the second compartment, said aperture including means to prevent the egress of powdered medicament from the used blister compartment in to the unused blister compartment through the aperture. The means may be a brush or elastomeric element.

In one embodiment, the dividing wall is configured so that it extends across said space between said sidewalls of the inhaler to prevent passage of powdered dose between the unused and used blister compartments.

In another embodiment, there are a plurality of dividing walls between the unused and used blister compartments, each dividing wall having an aperture for the passage of the blister strip from the unused to the used blister compartment. The aperture or opening in each dividing can then be positioned in staggered relationship to each other such that the blister strip follows a curved or tortuous path between the unused blister strip compartment and the used blister strip compartment.

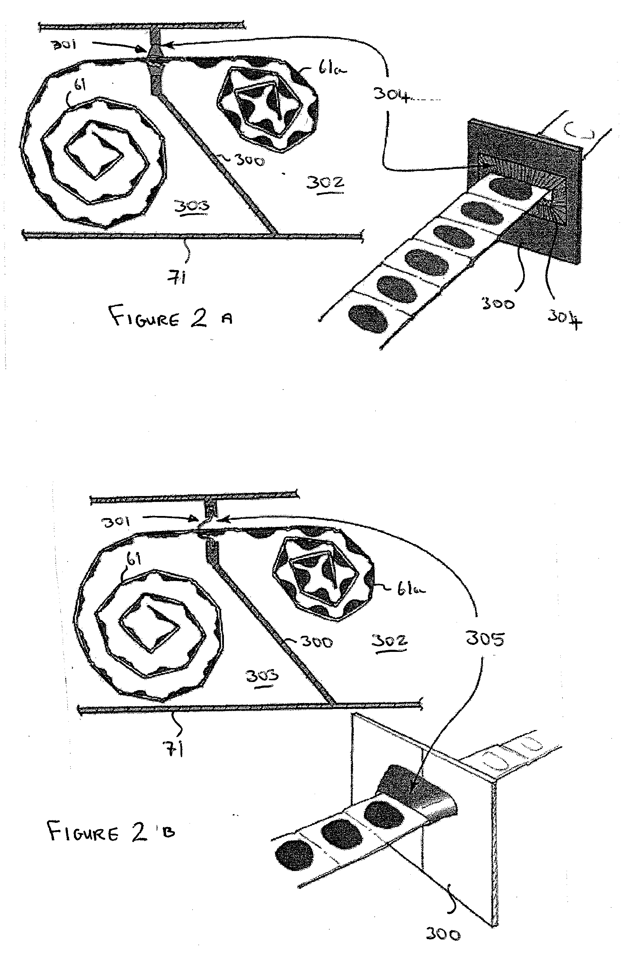

Embodiments of the invention will now be described, by way of example only, with reference to Figure 2 of the accompanying drawings, in which:-
FIGURE 1a and 1b are side sectional views of a conventional inhalation device to show how the blisters of a strip are sequentially moved into alignment with a blister piercing station by movement of an actuator from the position shown in Figure 1a to the position shown in Figure 1b which drives an indexing wheel. A piercing head on the actuator pierces the lid of an aligned blister when the actuator is returned to its normal position as shown in Figure 1a, and;
FIGURE 2a and 2b are partial enlarged sectional views of a portion of an inhaler according to the invention in which the used and unused blisters are contained in separate chambers by a dividing wall.

Reference is made throughout this specification to both "unused" and "used" blisters. It will be appreciated that "unused" blisters refer to those blisters that have not passed the blister piercing station and which remain intact with the dose contained therein. "Used" blisters refer to those blisters which have passed the blister piercing station in response to movement of the actuator by a user and which have been pierced to enable access to the dose contained therein to be obtained. Although in general, a "used" blister refers to a blister from which a dose has been inhaled, it should also be taken to include blisters which have passed the blister piercing station and have been pierced but which still contain either some or all of the dose contained therein. This may happen, for example, when a user moves the actuator to move the blister strip without inhaling the dose from a previously pierced blister.

To overcome the problem of contamination of unused blisters with residual powdered dose, the Applicants have proposed the provision of a dividing wall so as to separate the interior of the housing into a unused blister chamber and, a used blister chamber. This wall constrains any residual powder within the used blister portion of the housing. In one embodiment, the dividing wall is moulded integrally with the housing.

A partial section of an inhalation device according to an embodiment of the invention is illustrated in Figures 2a and 2b and from which it can be seen that the housing 71 is divided into two compartments by a fixed dividing wall 300. An aperture 301 is provided in the wall 300 through which the blister strip may pass from an unused blister compartment 302 into the used blister compartment 303. The edges of the aperture may be provided with a flexible element or seal such as a brush 304 (see Figure 2a) or elastomeric insert 305 (see Figure 2b) to reduce the amount of powder that can pass back through the aperture 301 from the used to the unused blister compartments 302,303.

It will be appreciated that a flexible element or seal around the aperture through which the blister strip passes from the unused blister strip compartment to the used blister strip compartment is not essential. Instead, the aperture may be made as small as possible so that it is a close fit around the strip without unduly interfering with movement of the strip through the aperture, thereby preventing or reducing the passage of powder between compartments through the aperture.

It is also envisaged that there can be more than one dividing wall, each dividing wall having an aperture therein through which the strip passes from one compartment to another. The apertures may be aligned with each other or be staggered so that the strip follows a curved or tortuous path through the apertures. As residual powder then has to pass through several apertures between compartments, the chances of contamination of the unused blister chamber are further reduced.

It will be appreciated that the inhaler of the invention may be either a passive or active device. In a passive device, the dose is entrained in a flow of air caused when the user inhales through the mouthpiece. However, in an active device, the inhaler would include means for generating a pressurised flow of gas or air through the blister to entrain the dose and carry it out of the blister through the mouthpiece and into the user's airway. In one embodiment, the inhaler may be provided with a source of pressurised gas or air within the housing.

A variety of medicaments may be administered alone by using inhalers of the invention. Such medicaments include those that are suitable for the treatment of asthma, chronic obstructive pulmonary diseases (COPD), respiratory infections, rhinitis, allergic rhinitis, nasal diseases and disorders; general and specific conditions, and systemic diseases with the lung or nasal cavity as the site of delivery. Such medicaments include, but are not limited to, β₂-agonists, eg carmoterol, fenoterol, formoterol, levalbuterol, pirbuterol, reproterol, metaproterenol, rimiterol, salbutamol, salmeterol, indacaterol, terbutaline, orciprenaline, clenbuterol, bambuterol, procaterol, broxaterol, picumeterol, and bitolterol; non-selective β-stimulants such as ephedrine and isoprenaline; phosphodiesterase (PDE) inhibitors, eg methylxanthines, theophylline, aminophylline, choline theophyllinate, and selective PDE isoenzyme inhibitors, PDE 3 inhibitors, eg milrinone and motapizone; PDE 4 inhibitors, eg rolipram, cilomilast, roflumilast, oglemilast, and ONO 6126; PDE 3/4 inhibitors, eg zardaverine and tolafentrine; inducers of HDAC2 eg theophylline; anticholinergics including muscarinic receptor (M1, M2, and M3) antagonists eg atropine, hyoscine, glycopyrrolate, ipratropium, tiotropium, oxitropium, NVA237, pirenzepine, and telenzepine; mast cell stabilisers, eg cromoglycate and ketotifen; bronchial anti-inflammatory agents, eg nedocromil; steroids, eg beclometasone, dexamethasone, fluticasone, budesonide, flunisolide, rofleponide, triamcinolone, butixocort, mometasone, and ciclesonide; disease modifying agents such as methotrexate, leflunomide, teriflunomide, and hydroxychloroquine; histamine type 1 receptor antagonists, eg cetirizine, loratadine, desloratadine, fexofenadine, acrivastine, terfenadine, astemizole, azelastine, levocabastine, chlorpheniramine, promethazine, cyclizine, and mizolastine; antibacterial agents and agents for cystic fibrosis and/or tuberculosis treatment, eg Pseudomonas aeruginosa infection vaccines (eg Aerugen®), mannitol, denufosol, glutathione, N-acetylcysteine, amikacin duramycin, gentamycin, tobramycin, dornase alfa, alpha 1-antitrypsin, heparin, dextran, capreomycin, vancomycin, meropenem, ciprofloxacin, piperacillin, and rifampicin; mucolytic agents for the treatment of COPD and cystic fibrosis, eg N-acetylcysteine, and ambroxol; histamine type 2 receptor antagonists; tachykinin neurokinin antagonists; triptans, eg almotriptan, rizatriptan, naratriptan, zolmitriptan, sumatritpan, eletriptan, and frovatriptan; neurological agents eg apomorphine, dronabinol, dihydroergotamine, and loxapine; antiviral agents eg foscarnet, acyclovir, famciclovir, valacyclovir, ganciclovir, cidofovir; amantadine, rimantadine; ribavirin; zanamivir and oseltamavir and pleconaril, protease inhibitors (eg ruprintrivir, indinavir, nelfinavir, ritonavir, and saquinavir), nucleoside reverse transcriptase inhibitors (eg didanosine, lamivudine, stavudine, zalcitabine, and zidovudine), and non-nucleoside reverse transcriptase inhibitors (eg nevirapine and efavirenz); α-1/α-2 adrenoceptor agonists, eg propylhexedrine, phenylephrine, phenylpropanolamine, ephedrine, pseudoephedrine, naphazoline, oxymetazoline, tetrahydrozoline, xylometazoline, tramazoline, and ethylnorepinephrine; platelet aggregation inhibitors/anti-inflammatory agents, eg bemiparin, enoxaparin, heparin; anti-infectives, eg cephalosporins, penicillins, tetracyclines, macrolides, beta-lactams, flouroquinolones, streptomycin, sulphonamides, aminoglycosides (eg tobramycin), doripenem, pentamidine, colistimethate, and aztreonam; agents for sexual health, sexual dysfunction including premature ejaculation; eg. apomorphine, VR776, agents that acts via 5HT- and noradrenergic-mediated pathways in the brain, leuprolide, and PDE 5 inhibitors eg, sildenafil, tadalafil, and vardenafil; leukotriene modifiers, eg zileuton, fenleuton, tepoxalin, montelukast, zafirlukast, ontazolast, ablukast, pranlikast, verlukast, and iralukast; inducible nitric oxide synthase (iNOS) inhibitors; antifungals, eg amphotericin B, natamycin, and nystatin; analgesics, eg codeine, dihydromorphine, ergotamine, fentanyl, cannabinoids, and morphine; anxiolytic/antidepressive agents, eg benzodiazepines and benzodiazepine derivatives, diazepam, midazolam, chlordiazepoxide, lorazepam, oxazepam, clobazam, alprazolam, clonazepam, flurazepam, zolazepam; tryptase and elastase inhibitors; beta-2 integrin antagonists; adenosine receptor agonists or antagonists, eg adenosine 2α agonists; calcium channel blockers, eg gallopamil, and diltiazem; prostacyclin analogues, eg iloprost; endothelin-receptor antagonists, eg LU-135252; cytokine antagonists, eg chemokine antagonists and inhibitors and modifiers of cytokine synthesis including modifiers and inhibitors of the pro-inflammatory transcription factor, NFkB; interleukins and inhibitors of interleukins, eg aldesleukin; therapeutic proteins and peptides, eg insulin, insulin aspart, insulin glulisine; insulin lispro, neutral, regular and soluble insulins, isophane insulins, insulin zinc, protamine zinc insulin, insulin analogues, acylated insulin, insulin glargine, insulin detemir, glucagon, glucagon-like peptides, and exendins; enzymes, eg dornase alfa; systemically active macromolecules, eg human growth hormone, leuprolide, alpha-interferon, growth factors (eg insulin-like growth factor type 1), hormones, eg epinephrine, testosterone, and parathyroid hormone and analogues (eg Ostabolin-C);; osteoporosis agents, eg bisphosphonates; anticancer agents, eg anthracyclines, doxorubicin, idarubicin, epirubicin, methotrexate, taxanes, paclitaxel, docetaxel, ciplatin, vinca alkaloids, vincristine, and 5-fluorouracil; anticoagulants, eg blood factors and blood factor constructs, eg FVIII-Fc and FIX-Fc;, eg FV111-Fc; immunomodulators, eg cyclosporine, sirolimus, and tacrolimus; antiproliferative immunosuppressants, eg azathioprine, and mycophenolate mofetil; cytokines (eg interferons, interferon β, interleukins, and interleukin antagonists and inhibitors); nucleic acids; vaccines, eg flumist; anti-obesity agents; diagnostics and gene therapies. It will be clear to a person skilled in the art that, where appropriate, the medicaments may be linked to a carrier molecule or molecules and/or used in the form of prodrugs, salts, as esters, or as solvates to optimise the activity and/or stability of the medicament.

Inhalers according to the invention may also be used to deliver combinations of two or more different medicaments. Specific combinations of two medicaments which may be mentioned include combinations of steroids and β₂-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; and mometasone and formoterol. Specifically inhalers according to the invention may also be used to deliver combinations of three different medicaments.

It will be clear to a person skilled in the art that, where appropriate, the medicaments may be linked to a carrier molecule or molecules and/or used in the form of prodrugs, salts, as esters, or as solvates to optimise the activity and/or stability of the medicament.

It is also envisaged that the pharmaceutical composition may comprise one or more, preferably one, anticholinergic **1**, optionally in combination with a pharmaceutically acceptable excipient.

The anticholinergic **1** can be selected from the group consisting of
a) tiotropium salts **1a,**
b) compounds of formula **1c** 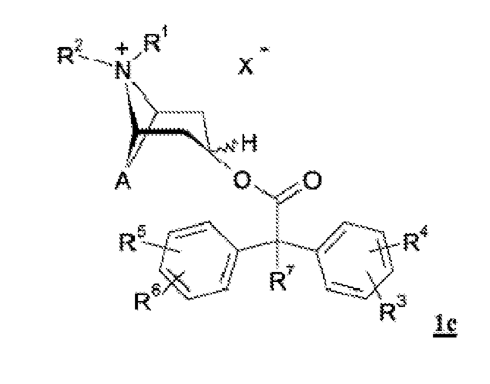 wherein
   A denotes a double-bonded group selected from among 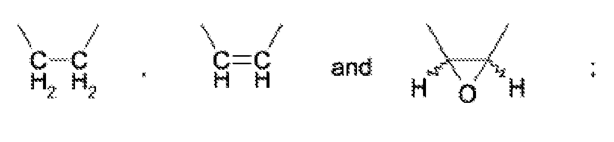 X⁻ denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,
   R¹ and R² which may be identical or different denote a group selected from among methyl, ethyl, n-propyl and iso-propyl, which may optionally be substituted by hydroxy or fluorine, preferably unsubstituted methyl;
   R³, R⁴, R⁵ and R⁶, which may be identical or different, denote hydrogen, methyl, ethyl, methyloxy, ethyloxy, hydroxy, fluorine, chlorine, bromine, CN, CF₃ or NO₂;
   R⁷ denotes hydrogen, methyl, ethyl, methyloxy, ethyloxy, -CH₂-F, -CH₂-CH₂-F, -0-CH₂-F, -0-CH₂-CH₂-F, -CH₂-OH, -CH₂-CH₂-OH, CF₃, - CH₂-OMe, -CH₂-CH₂-OMe, -CH₂-OEt, -CH₂-CH₂-OEt, -O-COMe, -O-COEt, -Q-COCF₃, -Q-COCF₃, fluorine, chlorine or bromine;
c) compounds of formula **1d** 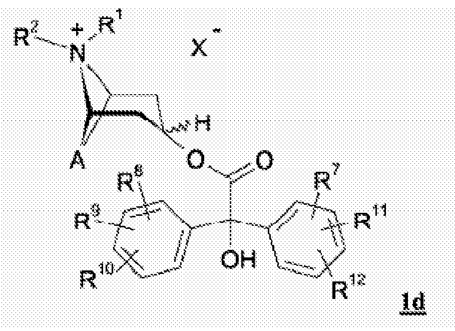 wherein
   A, X⁻, R¹ and R² may have the meanings as mentioned hereinbefore and wherein R⁷, R⁸, R⁹, R¹⁰, R¹¹ and R¹², which may be identical or different, denote hydrogen, methyl, ethyl, methyloxy, ethyloxy, hydroxy, fluorine, chlorine, bromine, CN, CF₃ or NO₂, with the proviso that at least one of the groups R⁷, R8, R9, R¹⁰, R¹¹ and R¹² is not hydrogen,
d) compounds of formula **1e** 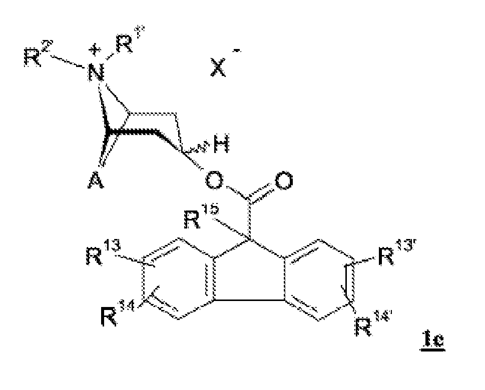 wherein A and X⁻ may have the meanings as mentioned hereinbefore, and wherein
   R¹⁵ denotes hydrogen, hydroxy, methyl, ethyl, -CF₃, CHF₂ or fluorine; R^{1'} and R^{2'} which may be identical or different denote C₁-C₅-alkyl which may optionally be substituted by C₃-C₆-cycloalkyl, hydroxy or halogen, or
   R^{1'} and R^{2'} together denote a -C₃-C₅-alkylene-bridge;
   R¹³, R¹⁴, R^{13'} and R^{14'} which may be identical or different denote hydrogen, -C₁-C₄-alkyl, -C₁-C₄-alkyloxy, hydroxy, -CF₃, -CHF₂, CN, NO₂ or halogen,
e) compounds of formula **1f** 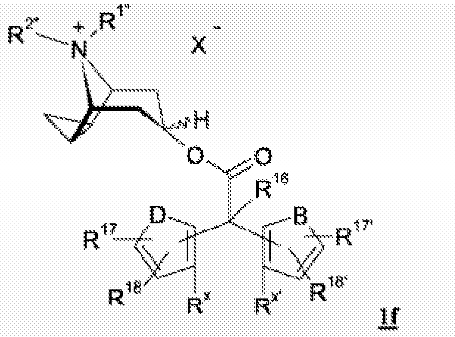 wherein X may have the meanings as mentioned hereinbefore, and wherein
   D and B which may be identical or different, preferably identical, denote -O, -S, -NH, -CH₂, -CH=CH, or -N(C₁-C₄-alkyl)-;
   R¹⁶ denotes hydrogen, hydroxy, -C₁-C₄-alkyl, -C₁-C₄ -alkyloxy, -C₁- C₄ - alkylene-Halogen, -O-C₁ -C₄ alkylene-halogen, -C₁ -C₄-alkylene-OH, -CF₃ , CHF₂, -C₁ -C₄-alkylene-C₁ -C₄ alkyloxy, -O- COC₁ -C₄-alkyl, -O-COC₁ -C₄ -alkylene-halogen, -C₁-C₄-alkylene-C₃-C₆-cycloalkyl, -O-COCF₃ or halogen;
   R^{1"} and R^{2"} which may be identical or different, denote -C₁ -C₅-alkyl, which may optionally be substituted by -C₃-C₆-cycloalkyl, hydroxy or halogen, or
   R^{1"} and R^{2"} together denote a -C3-C5-alkylene bridge; R¹⁷, R¹⁸, R^{17'} and R^{18'}, which may be identical or different, denote hydrogen, C₁-C₄-alkyl, C₁-C₄-alkyloxy, hydroxy, -CF₃, -CHF₂, CN, NO₂ or halogen;
   R^{x} and R^{x'} which may be identical or different, denote hydrogen, C₁-C₄-alkyl, C₁-C₄-alkyloxy, hydroxy, -CF₃, -CHF₂, CN, NO₂ or halogen or
   R^{x} and R^{x'} together denote a single bond or a bridging group selected from among the bridges -O, -S, -NH, -CH₂, -CH₂-CH₂-, -N(C₁-C₄-alkyl), -CH(C₁-C₄-alkyl)- and -C(C₁-C₄-alkyl)₂, and
f) compounds of formula **1g** 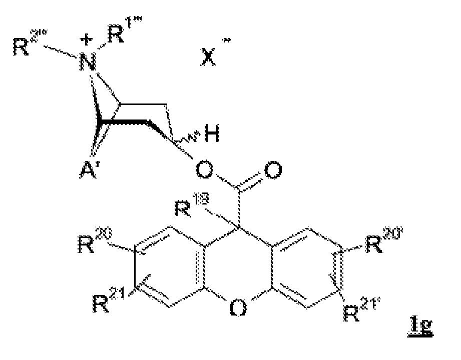
wherein X⁻ may have the meanings as mentioned hereinbefore, and wherein A' denotes a double-bonded group selected from among 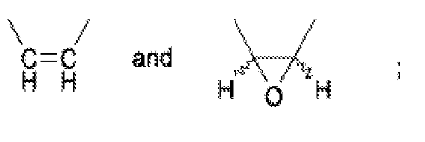 R¹⁹ denotes hydroxy, methyl, hydroxymethyl, ethyl, -CF₃, CHF₂ or fluorine; R^{1"'} and R^{2"'} which may be identical or different denote C₁-C₅-alkyl which may optionally be substituted by C₃-C₆-cycloalkyl, hydroxy or halogen, or
R^{1"'} and R^{2"'} together denote a -C₃-C₅-alkylene-bridge; R²⁰, R²¹, R^{20'} and R^{21'} which may be identical or different denote hydrogen, - C₁-C₄-alkyl, -C₁-C₄-alkyloxy, hydroxy, -CF₃, -CHF₂, CN, NO₂ or halogen.

The compounds of formula **1c** are known in the art (WO 02/32899).

In a preferred embodiment of the invention the method comprises administration of compounds of formula **1c**, wherein
X⁻ denotes bromide;
R¹ and R² which may be identical or different denote a group selected from methyl and ethyl, preferably methyl;
R³, R⁴, R⁵ and R⁶, which may be identical or different, denote hydrogen, methyl, methyloxy, chlorine or fluorine;
R⁷ denotes hydrogen, methyl or fluorine, optionally together with a pharmaceutically acceptable excipient.

Of particular importance are compounds of general formula **1c,** wherein A denotes a double-bonded group selected from among

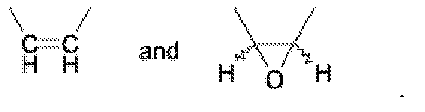

The compounds of formula **1c,** may optionally be administered in the form of the individual optical isomers, mixtures of the individual enantiomers or racemates thereof.

Of particular importance within a method according to the invention are the following compounds of formula **1c:**
tropenol 2,2-diphenylpropionic acid ester methobromide,
scopine 2,2-diphenylpropionic acid ester methobromide,
scopine 2-fluoro-2,2-diphenylacetic acid ester methobromide and
tropenol 2-fluoro-2,2-diphenylacetic acid ester methobromide.

The compounds of formula **1d** are known in the art (WO 02/32898).

In a preferred embodiment of the invention the method comprises administration of compounds of formula **1d,** wherein
A denotes a double-bonded group selected from among 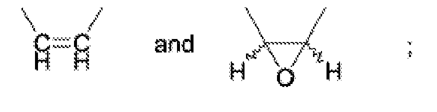 X⁻ denotes bromide;
R¹ and R² which may be identical or different denote methyl or ethyl, preferably methyl;
R⁷, R⁸, R⁹, R¹⁰, R¹¹ and R¹², which may be identical or different, denote hydrogen, fluorine, chlorine or bromine, preferably fluorine with the proviso that at least one of the groups R⁷, R⁸, R⁹, R¹⁰, R¹¹ and R¹² not hydrogen, optionally together with a pharmaceutically acceptable excipient.

Of particular importance within the method according to the invention are the following compounds of formula **1d**:
tropenol 3,3',4,4'-tetrafluorobenzilic acid ester methobromide,
scopine 3,3',4,4'-tetrafluorobenzilic acid ester methobromide,
scopine 4,4'-difluorobenzilic acid ester methobromide,
tropenol 4,4'-difluorobenzilic acid ester methobromide,
scopine 3,3'-difluorobenzilic acid ester methobromide, and
tropenol 3,3'-difluorobenzilic acid ester methobromide.

The pharmaceutical compositions according to the invention may contain the compounds of formula **1d** optionally in the form of the individual optical isomers, mixtures of the individual enantiomers or racemates thereof.

The compounds of formula **1e** are known in the art (WO 03/064419).

In a preferred embodiment of the invention the method comprises administration of compounds of formula **1e**, wherein
A denotes a double-bonded group selected from among 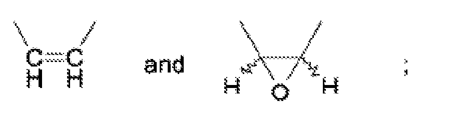 X⁻ denotes an anion selected from among chloride, bromide and methanesulphonate, preferably bromide;
R¹⁵ denotes hydroxy, methyl or fluorine, preferably methyl or hydroxy; R^{1'} and R^{2'} which may be identical or different represent methyl or ethyl, preferably methyl;
R¹³, R¹⁴, R^{13'} and R^{14'} which may be identical or different represent hydrogen, - CF₃, -CHF₂ or fluorine, preferably hydrogen or fluorine, optionally together with a pharmaceutically acceptable excipient.

In another preferred embodiment of the invention the method comprises administration of compounds of formula **1e,** wherein A denotes a double-bonded group selected from among 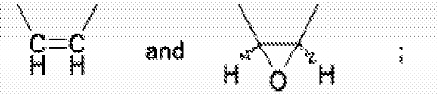 X⁻ denotes bromide;
R¹⁵ denotes hydroxy or methyl, preferably methyl; R^{1'} and R^{2'} which may be identical or different represent methyl or ethyl, preferably methyl;
R¹³, R¹⁴, R^{13'} and R^{14'} which may be identical or different represent hydrogen or fluorine, optionally together with a pharmaceutically acceptable excipient.

Of particular importance within the method according to the invention are the following compounds of formula **1e:**
tropenol 9-hydroxy-fluorene-9-carboxylate methobromide ;
tropenol 9-fluoro-fluorene-9-carboxylate methobromide;
scopine 9-hydroxy-fluorene-9-carboxylate methobromide;
scopine 9-fluoro-fluorene-9-carboxylate methobromide;
tropenol 9-methyl-fluorene-9-carboxylate methobromide;
scopine 9-methyl-fluorene-9-carboxylate methobromide.

The pharmaceutical compositions according to the invention may contain the compounds of formula **1e** optionally in the form of the individual optical isomers, mixtures of the individual enantiomers or racemates thereof.

The compounds of formula **1f** are known in the art (WO 03/064418).

In another preferred embodiment of the invention the method comprises administration of compounds of formula **1f** wherein
X denotes chloride, bromide, or methanesulphonate, preferably bromide; D and B which may be identical or different, preferably identical, denote -O, -S, -NH or -CH=CH-;
R¹⁶ denotes hydrogen, hydroxy, -C₁-C₄-alkyl, -C₁ -C₄ alkyloxy, -CF₃, -CHF₂, fluorine, chlorine or bromine;
R^{1"} and R^{2"} which may be identical or different, denote C₁-C₄-alky, which may optionally be substituted by hydroxy, fluorine, chlorine or bromine, or
R^{1"} and R^{2"} together denote a -C₃-C₄-alkylene-bridge;
R¹⁷, R¹⁸, R^{17'} and R^{18'}, which may be identical or different, denote hydrogen, C₁-C₄ -alkyl, C₁-C₄-alkyloxy, hydroxy, -CF₃, -CHF₂, CN, NO₂, fluorine, chlorine or bromine;
R^{x} and R^{x'} which may be identical or different, denote hydrogen, C₁-C₄-alkyl, C₁-C₄-alkyloxy, hydroxy, -CF₃, -CHF₂, CN, NO₂, fluorine, chlorine or bromine or
R^{x} and R^{x'} together denote a single bond or a bridging group selected from among the bridges -O, -S, -NH- and -CH₂-, optionally together with a pharmaceutically acceptable excipient.

In another preferred embodiment of the invention the method comprises administration of compounds of formula **1f,** wherein
**X⁻** denotes chloride, bromide, or methanesulphonate, preferably bromide; D and B which may be identical or different, preferably identical, denote -S or -CH=CH-;
R¹⁶ denotes hydrogen, hydroxy or methyl;
R^{1"} and R^{2"} which may be identical or different, denote methyl or ethyl;
R¹⁷, R¹⁸, R^{17'} and R^{18'}, which may be identical or different, denote hydrogen, - CF₃ or fluorine, preferably hydrogen;
R^{x} and R^{x'} which may be identical or different, denote hydrogen, -CF₃ or fluorine, preferably hydrogen or
R^{x} and R^{x'} together denote a single bond or the bridging group -O-, optionally together with a pharmaceutically acceptable excipient.

In another preferred embodiment of the invention the method comprises administration of compounds of formula If wherein
X⁻ denotes bromide;
D and B denote -CH=CH-;
R¹⁶ denotes hydrogen, hydroxy or methyl;
R^{1"} and R^{2"} denote methyl;
R¹⁷, R¹⁸, R^{17'} and R^{18'}, which may be identical or different, denote hydrogen or fluorine, preferably hydrogen;
R^{x} and R^{x'} which may be identical or different, denote hydrogen or fluorine, preferably hydrogen or
R^{x} and R^{x'} together denote a single bond or the bridging group -O-, optionally together with a pharmaceutically acceptable excipient.

Of particular importance within the method according to the invention are the following compounds of formula **1f**:
cyclopropyltropine benzilate methobromide;
cyclopropyltropine 2,2-diphenylpropionate methobromide;
cyclopropyltropine 9-hydroxy-xanthene-9-carboxylate methobromide;
cyclopropyltropine 9-methyl-fluorene-9-carboxylate methobromide;
cyclopropyltropine 9-methyl-xanthene-9-carboxylate methobromide;
cyclopropyltropine 9-hydroxy-fluorene-9-carboxylate methobromide;
cyclopropyltropine methyl 4,4'-difluorobenzilate methobromide.

The pharmaceutical compositions according to the invention may contain the compounds of formula **1f** optionally in the form of the individual optical isomers, mixtures of the individual enantiomers or racemates thereof.

The compounds of formula **1g** are known in the art (WO 03/064417).

In another preferred embodiment of the invention the method comprises administration of compounds of formula **1g** wherein A' denotes a double-bonded group selected from among 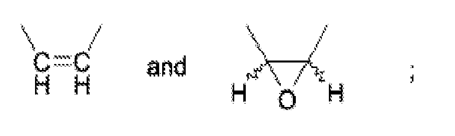 X⁻ denotes chloride, bromide or methanesulphonate, preferably bromide;
R¹⁹ denotes hydroxy or methyl;
R^{1"'} and R^{2"'} which may be identical or different represent methyl or ethyl, preferably methyl;
R²⁰, R²¹, R^{20'} and R^{21'} which may be identical or different represent hydrogen, - CF₃, -CHF₂ or fluorine, preferably hydrogen or fluorine, optionally together with a pharmaceutically acceptable excipient.

In another preferred embodiment of the invention the method comprises administration of compounds of formula **1g** wherein A' denotes a double-bonded group selected from among 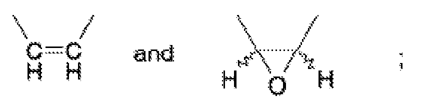 X⁻ denotes bromide;
R¹⁹ denotes hydroxy or methyl, preferably methyl;
R^{1"'} and R^{2"'} which may be identical or different represent methyl or ethyl, preferably methyl;
R³, R⁴, R^{3'} and R^{4'} which may be identical or different represent hydrogen or fluorine, optionally together with a pharmaceutically acceptable excipient.

Of particular importance within the method according to the invention are the following compounds of formula **1g**:
tropenol 9-hydroxy-xanthene-9-carboxylate methobromide;
scopine 9-hydroxy-xanthene-9-carboxylate methobromide;
tropenol 9-methyl-xanthene-9-carboxylate methobromide;
scopine 9-methyl-xanthene-9-carboxylate methobromide;
tropenol 9-ethyl-xanthene-9-carboxylate methobromide;
tropenol 9-difluoromethyl-xanthene-9-carboxylate methobromide;
scopine 9-hydroxymethyl-xanthene-9-carboxylate methobromide.

The pharmaceutical compositions according to the invention may contain the compounds of formula **1g** optionally in the form of the individual optical isomers, mixtures of the individual enantiomers or racemates thereof.

The alkyl groups used, unless otherwise stated, are branched and unbranched alkyl groups having 1 to 5 carbon atoms. Examples include: methyl, ethyl, propyl or butyl. The groups methyl, ethyl, propyl or butyl may optionally also be referred to by the abbreviations Me, Et, Prop or Bu. Unless otherwise stated, the definitions propyl and butyl also include all possible isomeric forms of the groups in question. Thus, for example, propyl includes n- propyl and iso-propyl, butyl includes iso-butyl, sec. butyl and tert. -butyl, etc.

The cycloalkyl groups used, unless otherwise stated, are alicyclic groups with 3 to 6 carbon atoms. These are the cyclopropyl, cyclobutyl, cyclopentyl and cyclohexyl groups. According to the invention cyclopropyl is of particular importance within the scope of the present invention.

The alkylene groups used, unless otherwise stated, are branched and unbranched double- bonded alkyl bridges with 1 to 5 carbon atoms. Examples include: methylene, ethylene, propylene or butylene.

The alkylene-halogen groups used, unless otherwise stated, are branched and unbranched double-bonded alkyl bridges with 1 to 4 carbon atoms which may be mono-, di- or trisubstituted, preferably disubstituted, by a halogen. Accordingly, unless otherwise stated, the term alkylene-OH groups denotes branched and unbranched double-bonded alkyl bridges with 1 to 4 carbon atoms which may be mono-, di- or trisubstituted, preferably monosubstituted, by a hydroxy.

The alkyloxy groups used, unless otherwise stated, are branched and unbranched alkyl groups with 1 to 5 carbon atoms which are linked via an oxygen atom. The following may be mentioned, for example: methyloxy, ethyloxy, propyloxy or butyloxy. The groups methyloxy, ethyloxy, propyloxy or butyloxy may optionally also be referred to by the abbreviations MeO, EtO, PropO or BuO. Unless otherwise stated, the definitions propyloxy and butyloxy also include all possible isomeric forms of the groups in question. Thus, for example, propyloxy includes n-propyloxy and iso-propyloxy, butyloxy includes iso-butyloxy, sec. butyloxy and tert. -butyloxy, etc. The word alkoxy may also possibly be used within the scope of the present invention instead of the word alkyloxy. The groups methyloxy, ethyloxy, propyloxy or butyloxy may optionally also be referred to as methoxy, ethoxy, propoxy or butoxy.

The alkylene-alkyloxy groups used, unless otherwise stated, are branched and unbranched double-bonded alkyl bridges with 1 to 5 carbon atoms which may be mono-, di- or trisubstituted, preferably monosubstituted, by an alkyloxy group.

The -O-CO-alkyl groups used, unless otherwise stated, are branched and unbranched alkyl groups with 1 to 4 carbon atoms which are bonded via an ester group. The alkyl groups are bonded directly to the carbonylcarbon of the ester group. The term -O-CO-alkyl-halogen group should be understood analogously. The group -O-CO-CF₃ denotes trifluoroacetate.

Within the scope of the present invention halogen denotes fluorine, chlorine, bromine or iodine. Unless otherwise stated, fluorine and bromine are the preferred halogens. The group CO denotes a carbonyl group.

One aspect of the invention is directed to an inhalation device, in which the plural of doses are contained in one reservoir. In another aspect of the invention, the inhalation device comprises the plural of doses in a multi-dose blister pack. In another aspect of the invention the inhalation device comprises the multi-dose blister pack in form of blister strip.

The inhalation device according to the invention comprises the compounds of formula 1 preferably in admixture with a pharmaceutically acceptable excipient to form a powder mixture. The following pharmaceutically acceptable excipients may be used to prepare these inhalable powder mixtures according to the invention: monosaccharides (e.g. glucose or arabinose), disaccharides (e.g. lactose, saccharose, maltose, trehalose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose and trehalose are the particularly preferred excipients, while lactose, preferably in form of its monohydrate is most particularly preferred.

The compounds of formula 1 may be used in the form of their racemates, enantiomers or mixtures thereof. The separation of enantiomers from the racemates may be carried out using methods known in the art (e.g. by chromatography on chiral phases, etc.).

Optionally, the inhalation device according to the invention contains plural of doses of a medicament in powder form that contains, beside one compound of formula 1, another active ingredient.

Preferably the additional active ingredient is a beta₂ agonists **2** which is selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-Hydroxy-2-(4-hydroxy-3- hydroxymethyl-phenyl)-ethylamino]-hexyloxy}-butyl)-benzenesulfoneamide, 5-[2-(5,6- Diethyl-indan-2-ylamino)-1-hydroxy-ethyl]-8-hydroxy-1H-quinolin-2-one , 4-hydroxy-7- [2- { [2- {[3 -(2-phenylethoxy)propyl] sulphonyl} ethyl] -amino} ethyl] -2(3H)-benzothiazolone , 1 -(2-fluoro-4-hydroxyphenyl)-2-[4-(1-benzimidazolyl)-2-methyl-2-butylamino]ethanol , 1 - [3 - (4-methoxybenzyl-amino)-4-hydroxyphenyl] -2- [4-(1-benzimidazolyl)-2-methyl-2- butylamino]ethanol , 1 -[2H-5-hydroxy-3-oxo-4H-1,4-benzoxazin-8-yl]-2-[3-(4-N,N- dimethylaminophenyl)-2-methyl-2-propylamino]ethanol , 1-[2H-5-hydroxy-3-oxo-4H-1,4-benzoxazin-8-yl]-2-[3-(4-methoxyphenyl)-2-methyl-2-propylamino]ethanol , 1 -[2H-5- hydroxy-3-0X0-4H-1 ,4-benzoxazin-8-yl] -2- [3 -(4-n-butyloxyphenyl)-2-methyl-2- propylamino]ethanol , 1 - [2H-5-hydroxy-3 - oxo-4H- 1 ,4-benzoxazin-8-yl] -2- {4- [3 -(4- methoxyphenyl)-1,2,4-triazol-3-yl]-2-methyl-2-butylamino}ethanol , 5-hydroxy-8-(1- hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1 -(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred beta₂ agonists **2** are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4- {6-[2-Hydroxy-2-(4-hydroxy-3-hydroxymethyl-phenyl)- ethylamino]-hexyloxy} -butyl)-benzenesulfoneamide, 5-[2-(5,6-Diethyl-indan-2-ylamino)- 1-hydroxy-ethyl]-8-hydroxy-1H-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro- 4-hydroxyphenyl)-2-[4-(1-benzimidazolyl)-2-methyl-2-butylamino]ethanol , 1 -[3-(4-methoxybenzyl-amino)-4-hydroxyphenyl] -2- [4-( 1 -benzimidazolyl)-2-methyl-2- butylamino]ethanol, 1-[2H-5-hydroxy-3-oxo-4H-1,4-benzoxazin-8-yl]-2-[3-(4-N,N- dimethylaminophenyl)-2-methyl-2-propylamino]ethanol , 1-[2H-5-hydroxy-3-oxo-4H-1,4- benzoxazin-8-yl]-2-[3-(4-methoxyphenyl)-2-methyl-2-propylamino]ethanol , 1-[2H-5-hydroxy-3 -OXO-4H- 1 ,4-benzoxazin-8-yl] -2- [3 -(4-n-butyloxyphenyl)-2-methyl-2-propylamino]ethanol , 1 - [2H-5-hydroxy-3 -oxo-4H- 1 ,4-benzoxazin-8-yl] -2-{4-[3 -(4- methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5- trifluormethylphenyl)-2-tert.-butylamino)ethanol and 1 -(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

More preferably, the betamimetics **2** used as within the compositions according to the invention are selected from among fenoterol, formoterol, salmeterol, 3-(4-{6-[2-Hydroxy- 2-(4-hydroxy-3-hydroxymethyl-phenyl)-ethylamino]-hexyloxy}-butyl)- benzenesulfoneamide, 5-[2-(5,6-Diethyl-indan-2-ylamino)-1-hydroxyethyl]-8-hydroxy- 1H-quinolin-2-one, 1 -[3-(4-methoxybenzyl-amino)-4-hydroxyphenyl]-2-[4-(1 - benzimidazolyl)-2-methyl-2-butylamino]ethanol , 1-[2H-5-hydroxy-3-oxo-4H-1,4- benzoxazin-8-yl]-2-[3-(4-N,N-dimethylaminophenyl)-2-methyl-2-propylamino]ethanol , 1 - [2H-5-hydroxy-3-oxo-4H-1,4-benzoxazin-8-yl]-2-[3-(4-methoxyphenyl)-2-methyl-2- propylamino]ethanol , 1-[2H-5-hydroxy-3-oxo-4H-1,4-benzoxazin-8-yl]-2-[3-(4-n- butyloxyphenyl)-2-methyl-2-propylamino]ethanol , 1-[2H-5-hydroxy-3-oxo-4H-1,4- benzoxazin-8-yl] -2- {4- [3 -(4-methoxyphenyl)- 1 ,2,4-triazol-3 -yl] -2-methyl-2- butylamino}ethanol, optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethylphenyl)-ethylamino]- hexyloxy}-butyl)-benzenesulfoneamide, and 5-[2-(5,6-Diethyl-indan-2-ylamino)-1-hydroxy-ethyl]-8-hydroxy-1H-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics **2** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4- difluorophenyl)salicylic acid or maleic acid. If desired, mixtures of the abovementioned acids may also be used to prepare the salts **2**.

According to the invention, the salts of the betamimetics **2** selected from among the hydrochloride, hydrobromide, sulphate, phosphate, fumarate, methanesulphonate, 4- phenylcinnamate, 5-(2.4-difluorophenyl)salicylate, maleate and xinafoate are preferred. Particularly preferred are the salts of 2 in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of 2 in the case of formoterol selected from the hydrochloride, sulphate and fumarate, of which the hydrochloride and fumarate are particularly preferred. Of exceptional importance according to the invention is formoterol fumarate.

Salts of salmeterol, formoterol, 3-(4-{6-[2-Hydroxy-2-(4-hydroxy-3-hydroxymethyl- phenyl)-ethylamino]-hexyloxy} -butyl)-benzenesulfoneamide, and 5-[2-(5,6-Diethyl-indan- 2-ylamino)-1-hydroxy-ethyl]-8-hydroxy-1H-quinolin-2-one, are preferably used as the betamimetics 2 according to the invention. Of particular importance according to the invention are salmeterol and formoterol salts. Any reference to the term betamimetics **2** also includes a reference to the relevant enantiomers or mixtures thereof. In the pharmaceutical compositions according to the invention, the compounds **2** may be present in the form of their racemates, enantiomers or mixtures thereof. The separation of the enantiomers from the racemates may be carried out using methods known in the art (e.g. by chromatography on chiral phases, etc.) If the compounds 2 are used in the form of their enantiomers, it is particularly preferable to use the enantiomers in the R configuration at the C-OH group.

Optionally, the inhalation device according to the invention contains plural of doses of a medicament in powder form, that contains beside one compound of formula **1** a steroid **3** as another active ingredient.

In such medicament combinations the steroid **3** is preferably selected from among prednisolone, prednisone , butixocortpropionate, RPR- 106541, flunisolide , beclomethasone , triamcinolone , budesonide , fluticasone, mometasone , ciclesonide , rofleponide , ST- 126 , dexamethasone , (S)-fluoromethyl 6α,9α-difluoro-17α-[(2- furanylcarbonyl)oxy] - 11 [beta]-hydroxy-16α-methyl-3 -oxo-androsta- 1 ,4-diene- 17β- carbothionate , (S)-(2-oxo-tetrahydro-furan-3S-yl)6α,9α-difluoro-11β-hydroxy- 16α- methyl-3 -oxo- 17α-propionyloxy-androsta- 1 ,4-diene- 17β-carbothionate, and etiprednol-dichloroacetate (BNP- 166), optionally in the form of the racemates, enantiomers or diastereomers thereof and optionally in the form of the salts and derivatives thereof, the solvates and/or hydrates thereof.

In particularly preferred medicament combinations the steroid **3** is selected from the group comprising flunisolide , beclomethasone , triamcinolone , budesonide , fluticasone , mometasone , ciclesonide , rofleponide , ST- 126 , dexamethasone , (S)-fluoromethyl 6α,9α-difluoro- 1 Ia- [(2-furanylcarbonyl)oxy] - 11 β-hydroxy-16α-methyl-3 -oxo-androsta- 1,4-diene-17β-carbothionate , (S)-(2-oxo-tetrahydro-furan-3S-yl)6α,9α-difluoro-11β- hydroxy- 16α-methyl-3 -oxo- 17α-propionyloxy-androsta- 1 ,4-diene- 17β-carbothionate , and etiprednol-dichloroacetate , optionally in the form of the racemates, enantiomers or diastereomers thereof and optionally in the form of the salts and derivatives thereof, the solvates and/or hydrates thereof.

In particularly preferred medicament combinations the steroid **3** is selected from the group comprising budesonide , fluticasone , mometasone , ciclesonide , (S)-fluoromethyl 6α,9α-difluoro- 1 Ia- [(2-furanylcarbonyl)oxy] - 11β-hydroxy-16α-methyl-3 -oxo-androsta- 1 ,A- diene-17β-carbothionate , and etiprednol-dichloroacetate , optionally in the form of the racemates, enantiomers or diastereomers thereof and optionally in the form of the salts and derivatives thereof, the solvates and/or hydrates thereof.

Any reference to steroids **3** includes a reference to any salts or derivatives, hydrates or solvates thereof which may exist. Examples of possible salts and derivatives of the steroids **3** may be: alkali metal salts, such as for example sodium or potassium salts, sulphobenzoates, phosphates, isonicotinates, acetates, propionates, dihydrogen phosphates, palmitates, pivalates or furcates.

Optionally, the inhalation device according to the invention contains plural of doses of a medicament on powder form, that contains beside one compound of formula **1** additionally both, one of the betamimetics **2** mentioned hereinbefore and one of the steroids **3** mentioned hereinbefore.

According to the invention, therefore, there may be provided a housing to receive a strip of blisters each containing a dose of medicament and means to sequentially move each blister into alignment with means for opening a blister to enable a user to inhale said dose, the inhaler having a first compartment to contain unused blisters and a second compartment to receive used blisters, the first and second compartments being separated by a fixed dividing wall, wherein each blister contains a pharmaceutical composition in powder form wherein the pharmaceutical composition comprises one or more, preferably one, compound of formula 1.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipients mentioned above. These finer excipients are also selected from the group of possible excipients listed hereinbefore. Finally, in order to prepare the inhalable powders according to the invention, micronised active substance I-, and optionally 2 and/or 3, preferably with an average particle size of 0.5 to 10µm, more preferably from 1 to 6µm, is added to the excipient mixture. Processes for producing the inhalable powders according to the invention by grinding and micronising and finally mixing the ingredients together are known from the prior art.

For the methods of preparing the pharmaceutical compositions in powder form reference may be made to the disclosure of WO 02/30390, WO 03/017970, or WO 03/017979 for example. The disclosure of WO 02/30390, WO 03/017970, and WO 03/017979 is herby incorporated by reference into the instant patent application in its entirety.

As an example, the pharmaceutical compositions according to the invention may be obtained by the method described below.

First, the excipient and the active substance are placed in a suitable mixing container. The active substance used has an average particle size of 0.5 to 10 µm, preferably 1 to 6 µm, most preferably 2 to 5 µm. The excipient and the active substance are preferably added using a sieve or a granulating sieve with a mesh size of 0.1 to 2 mm, preferably 0.3 to 1 mm, most preferably 0.3 to 0.6 mm. Preferably, the excipient is put in first and then the active substance is added to the mixing container. During this mixing process the two components are preferably added in batches. It is particularly preferred to sieve in the two components in alternate layers. The mixing of the excipient with the active substance may take place while the two components are still being added. Preferably, however, mixing is only done once the two components have been sieved in layer by layer.

If after being chemically prepared the active substance used in the process described above is not already obtainable in a crystalline form with the particle sizes mentioned earlier, it can be ground up into the particle sizes which conform to the above-mentioned parameters (so-called micronising).

Many modifications and variations of the invention falling within the terms of the following claims will be apparent to those skilled in the art and the foregoing description should be regarded as a description of the preferred embodiments of the invention only.

## Claims

1. An inhaler comprising a housing to receive a strip of blisters each containing a dose of medicament and means to sequentially move each blister into alignment with means for opening a blister to enable a user to inhale said dose, wherein the housing comprises a first compartment to contain unused blisters and a second compartment to receive used blisters, the first and second compartments being separated by a dividing wall.

2. An inhaler according to claim 1, wherein the dividing wall is fixed.

3. An inhaler according to claim 1 or claim 2, comprising an aperture in the dividing wall for the passage of the blister strip from the first compartment into the second compartment.

4. An inhaler according to claim 3, comprising means to prevent the egress of powdered medicament from the used blister compartment into the unused blister compartment through the aperture.

5. An inhaler according to claim 4, wherein said means comprises a brush extending at least partially across the aperture in the dividing wall.

6. An inhaler according to claim 5, wherein said means comprises an elastomeric element extending at least partially across the aperture in the dividing wall.

7. An inhaler according to any of claims 2 to 6, wherein there are a plurality of dividing walls between the unused and used blister compartments, each dividing wall having an aperture for the passage of the blister strip from the unused to the used blister compartment.

8. An inhaler according to claim 7, wherein the apeture in each dividing wall is positioned such that the blister strip follows a tortuous path between the unused blister strip compartment and the used blister strip compartment.

9. An inhaler according to any preceding claim, wherein the housing contains a coiled strip of blisters.
